# EUROPEAN PATENT SPECIFICATION

(11) **EP 2 978 481 B1**
(45) Date of publication and mention of the grant of the patent: **28.12.2016**
(21) Application number: 14712750.0
(22) Date of filing: 25.03.2014
(51) Int. Cl.: A61M 15/00, A61M 15/06, A24F 47/00

(54) **INHALER**
INHALATOR
INHALATEUR

(30) Priority: 26.03.2013 GB 201305496
(43) Date of publication of application: 03.02.2016
(73) Proprietor: Kind Consumer Limited, London EC1R 5AR (GB)
(72) Inventor: HEARN, Alex, London Greater London EC1R 5BX (GB); NYEIN, Khine Zaw, London SE3 9QA (GB)
(74) Representative: Boult Wade Tennant
(86) International application number: PCT/GB2014/050937
(87) International publication number: WO 2014/155091

(56) References cited:
- WO-A1-2011/015825
- WO-A1-2011/107737
- WO-A2-2004/041340
- WO-A2-2011/117580
- WO-A2-2012/045683
- DEHAO JU ET AL: "Effect of expansion chamber geometry on atomization and spray dispersion characters of a flashing mixture containing inerts. Part I. Numerical predictions and dual laser measurements", INTERNATIONAL JOURNAL OF PHARMACEUTICS, ELSEVIER BV, NL, vol. 432, no. 1, 23 April 2012 (2012-04-23), pages 23-31, XP028510743, ISSN: 0378-5173, DOI: 10.1016/J.IJPHARM.2012.04.065 [retrieved on 2012-04-28] cited in the application

## Description

The present invention relates to an inhaler. In particular, the invention relates to a novel design of outlet configuration designed to produce a particular particle size distribution from a reservoir of pressurised composition.

The invention has primarily been designed as a development to a simulated cigarette such as that disclosed in WO 2011/015825. However, it has broad applications in other types of inhaler such as a metered dose inhaler (MDI) of the type commonly used in asthma inhalers.

Aerosols are an attractive means of delivering drugs to patients when the site of action is the lungs themselves or for quick delivery of drugs to the brain. The particle size of aerosols is an important parameter to control when delivering an inhaled composition since the depth of penetration into the lungs increases with reducing particle size. It plays a significant role in determining the deposition profile of the aerosol in the respiratory system. It is known that larger particles (>10µm) are deposited in the mouth and upper thoracic region, whilst smaller particles (<10µm) have deposition distributions that are from the upper thoracic through to the alveolar region. Fine droplets (0.1µm < Dm < 1µm) have good alveolar deposition at between 1-5µm. Ultra-fine droplets (<0.1µm) are optimal for alveolar deposition from where drug molecules can be efficiently absorbed into the circulatory system, but are currently not feasibly produced in a portable device. This deposition distribution can be exploited to allow for effective delivery of pharmaceuticals, proteins, vaccines or, nicotine in the case of a simulated cigarette device. It is known that the D50 (mass-median-diameter, or average particle size by mass) of cigarette smoke is between 0.3-0.5µm for most main stream cigarettes. To be successful as a cigarette replacement, ideally a simulated cigarette would be able to reproduce this particle size.

WO 2004/022242 discloses an aerosol generating device wherein the generated droplet size lies between 0.5 and 2.5 µm. The droplet size is controlled by preferably increasing the exit velocity of a vapour that is generated by heating the formulation liquid source while it passes through a capillary sized flow pathway. The vapour, after exiting, is mixed with air to produce an aerosol.

US 5,957,124 discloses devices, packaging and methodology for creating aerosols with particle size ranging from 0.5 to 12 µm. The device comprises of collapsible containers containing the drug formulations, which when actuated, forces the formulation through a membrane having pores of 0.25 to 6 µm diameters. This membrane is aligned such that the formulation is forced from the containers into a channel through which the patient inhales air. Sufficient energy from the air (which may or may not be heated) is imparted to the formulation to induce particle size reduction. It incorporates a microprocessor into the device to obtain real-time measurements of inspiratory volume and flow rate for determination of a beginning point to force formulation through the pores.

WO 2008/151796 teaches an inhaler that produces an aerosol which has a mean particle size of 2-5µm. Also, the primary aim is to devise an inhaler that has a flow resistance of at least 60000Pa^{½}s/m³, which translates to a much higher draw resistance when compared to existing devices of a similar type.

US 7,293,559 discloses a device for creating an aerosol through the use of a focusing funnel to focus the liquid stream using a second fluid stream, leading to a mean particle size of 2µm upon exit of the device.

WO 2011/015825, our own earlier application, discloses an inhaler composed of a non-metered breath activated valve comprising a flow path in the form of a deformable tube extending from a reservoir (containing the formulation) to an outlet end. It discloses a clamping member which pinches the deformable tube when no suction is applied resulting in obstruction of flow. It releases the pinch to form an opening when suction is applied to provide uninterrupted flow from the reservoir to the outlet. However, the disclosure concerns flow control and makes the reference to the particle size generated using this design. Testing of a similar device is disclosed in Dehao Ju et al: "Effect of expansion chamber geometry on atomization and spray dispersion characters of a flashing mixture containing inerts. Part 1. Numerical predictions and dual laser measurements" International Journal of Pharmaceutics, Elsevier BV, NL, vol. 432, no. 1, 23 April 2012 (2012-04-23), pages 23-31, XP028510743, ISSN: 0378-5173, DOI: 10.1016/J. IJPHARM.2012.04.065.

According to the present invention there is provided an inhaler comprising a reservoir of pressurised inhalable composition, an outlet valve to control the flow of composition from the reservoir, the valve outlet orifice having a maximum height h, measured in the direction of opening when fully opened; an expansion chamber downstream of the valve having a length L and diameter D measured half way along the expansion chamber; and an exit orifice at the downstream end of the expansion chamber, the exit orifice having a length 1 and a
diameter d; wherein:
0.1 < h/d < 1.0
0.05 < h/D < 0.25
1 < D/d < 10
5 < L/D < 15
2 < 1/d < 3.
wherein the exit orifice is less than 3mm from an outlet end of the inhaler.

Such an arrangement is capable of delivering particles size distribution with a D50 of 0.5µm and can therefore produce the type of small particle size distributions that are to be found in cigarette smoke. However, the present invention achieves this simply by careful selection of the geometry of existing components. It does not, as with the prior art, require any additional features such as baffles or heat input. The avoidance of heating the composition is beneficial as it avoids degradation and problems associated with the risk of fumes.

A combination of the various parameters quoted above is one which has been arrived at after numerous tests required to identify the key geometrical relationships for producing the optimum particle size. All five of the parameters are inter-related. However, broadly speaking, the effect of the parameters is as follows.

The ratio h/d is important in promoting turbulent flow. Having an exit orifice, the diameter of which is equal to or greater than the valve outlet height ensures a homogenous pattern of bubbles dispersion which is favourable for droplet size reduction and the formation of a spray instead of a jet.

Preferably h/d is between 0.2 and 0.9 and more preferably substantially 0.5.

The ratio h/D is important in ensuring that there is sufficient volume for expansion of the formulation as it passes through the valve outlet orifice. If the diameter of the expansion chamber is too large, flow will be laminar thus preventing effective particle break up. Preferably h/D is between 0.05 and 0.25 and more preferably between 0.10 and 0.15.

The ratio D/d has an important role in maintaining a small droplet size exiting the device as ensuring that there is still sufficient volume for mixing whilst avoiding significant dead zones. Preferably, D/d is between 2 and 7 and more preferably between 3 and 5.

The ratio L/D effects the flow regime inside the expansion chamber. Sufficient volume is provided within the claimed range for the formulation to evaporate, re-circulate and form sufficiently sized bubbles to provide droplets of a small, uniform size upon exit of the outlet orifice. Preferably, the ratio L/D is between 6 and 13 and more preferably between 7 and 10.

The diameter D is specified as being measured at the mid point of the expansion chamber. This is because preferably, the expansion chamber tapers from the valve outlet orifice to the exit orifice at an included angle of between 0 and 30º, preferably between 9 and 10º and most preferably substantially 2º. This tapering prevents dead zones from forming in the expansion chamber and promotes a well-mixed system which is useful in maintaining a uniform aerosol.

The ratio 1/d is important in the formulation of a turbulent exit aerosol. By optimising its ratio, the droplet size can be decreased or increased.

Preferably, 0.05<h<1mm and more preferably 0.05<h<0.6mm. Preferably, 0.15<d<0.25mm. Preferably, 7.0<L<7.8mm. Preferably, 0.40<1<0.5mm.

The outlet valve may, for example, be a sliding gate valve member which opens to the required extend. However, preferably, the outlet valve is a pinch valve in which a valve element pinches a deformable tube with the outlet orifice height representing the maximum opening height at the pinch point. It is preferably a breath operated valve.

The deformable tube preferably also provides the expansion chamber and the exit orifice. This allows the profile of the droplet size of the emitted aerosol to be defined entirely by the dimensions of a single component. This is extremely useful in tuning the inhaler to the required particle size and also in producing inhalers offering a range of particle profiles whilst only having to change a single component in order to achieve these different sizes.

Preferably, the inhaler is configured so that the Reynolds number of the aerosol at the exit is between 1000 and 4000 and preferably between 1500 and 3000.

The inhaler may be an MDI, but is preferably a simulated cigarette. Preferably, the inhalable composition comprises nicotine and a propellant.

In order to further minimise impact between the composition and the inhaler downstream of the exit orifice, there is preferably a flared flow path with an angle of at least 10° between the exit orifice and the outlet end of the inhaler.

An example of an inhaler in accordance with the present invention will now be described with reference to the accompanying drawings, in which:
Figs. 1 and 2 are cross-sectional views of a prior art inhaler in closed and open configurations respectively;
Fig. 3 is a cross-section showing the geometry of the outlet valve of an inhaler according to the present invention; and
Fig. 4 is a cross-section showing the outlet end of the inhaler according to the present invention.

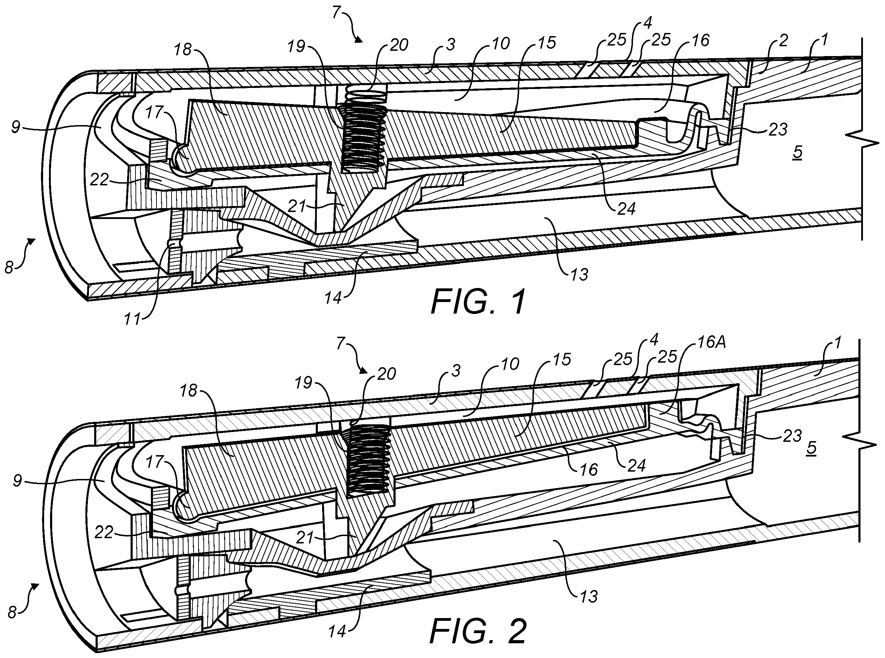

The basic design of a simulated cigarette which is an example of an inhaler that the present invention improves upon is shown in Figs. 1 and 2 which are taken from WO 2011/015825.

The device has a housing 1 made up of a main chassis 2 and a closure element 3 as shown in Fig. 1. This is held in place by label 4. Within the housing, there is a reservoir 5 containing the inhalable composition.

The breath-activated valve 7 is positioned between an outlet end 8 and the reservoir 5. The breath-activated valve is arranged so that, when a user sucks on the outlet end 8, the breath-activated valve 7 opens to allow the inhalable composition from the reservoir 5 to be inhaled.

The housing at the outlet end has two orifices. The first of these is the suction orifice 9 which communicates with a chamber 10 as will be described in greater detail below and the second is an outlet orifice 11 from which the inhalable composition dispensed is also described in more detail below.

An outlet path 13 is defined between the reservoir 5 and outlet orifice 11.

A portion of the outlet path 13 is provided by deformable tubular element 14. This tubular element is moved between the closed position shown in Fig. 1 and the open position shown in Fig. 2 by a mechanism which will now be described.

This mechanism comprises a pivotally mounted vane 15 and a membrane 16. The pivotally mounted vane has a pivot 17 at the end closest to the outlet end 8 and a central reinforcing rib 18 running along its length and tapering away from the outlet end. At around the midpoint, the vane 15 is provided with a recess 19 for receiving a spring 20 which biases it into the closed position shown in Fig. 1. Below the recess 19 is a jaw 21 having a triangular cross-section which is configured to apply the force provided from the vane 15 to the deformable tube 14 over a narrow area. The vane 15 is supported by the diaphragm 16 which is sealed to the housing at its ends 22, 23. This seals off the chamber 10 other than to the suction orifice 9.

The underside 24 of the membrane 16 is open to atmospheric pressure as a leakage path exists through the housing 1 which is not shown in the drawings as it extends around the outlet path 1 and is therefore not shown in the plane of Figs. 1 and 2.

When a user sucks on the outlet end 8 with the device in the configuration shown in Fig. 1, the suction is communicated by the suction orifice 9 to the chamber 10 through orifices 25 thereby lowering the pressure in this chamber. This causes the vane 15 to be lifted against the action of the spring 20 to the position shown in Fig. 2 deforming the diaphragm into the configuration shown in Fig. 2 and lifting the jaw 21 to allow the deformable tube to open, thereby allowing the inhalable composition from the reservoir 5 along outlet path 13 through the deformable tube 14 and out through the outlet orifice 11. The degree of suction applied by the user will determine the extent to which the vane 15 moves and therefore the amount of composition that the user receives. As soon as a user stops sucking, atmospheric pressure will return to the chamber 10 via the suction orifice 9 and the spring 20 will return the vane to the Fig. 1 position thereby pinching the tube 14 closed.

In a later example in WO 2011/015825, the outlet orifice 11 is provided in the same deformable tube component as is used for the deformable tubular element 14. A further example of such an element is disclosed in our later WO 2011/107737.

A further modification to the breath activated valve is disclosed in pending UK application 1215278.1. In this modification, there are no orifices 25 such that the chamber 10 is a blind chamber. Orifices are provided in the lower part of the cigarette to provide an air flow path over the underside of the membrane 16 which has an outlet at the outlet end 8. When a user sucks on this cigarette, there is a decrease in the pressure in the upper chamber and an increase in the pressure in the air flow path on the underside of the chamber to open the valve.

The present invention concerns an improvement in the valve geometry. As such, it is applicable to either of the above described flow paths. It is applicable to both arrangement shown in Figs. 1 and 2 where the outlet orifice is in a separate component from the deformable tubular element 14, or where they are combined into a single element as described above.

Further, although the invention is motivated an improvement to a simulated cigarette, it can be more broadly applied to other types of inhaler such as a metered dose inhaler. Also, whilst the size of the valve outlet orifice is an important parameter of the present invention, the valve may be operated by any known mechanism. It may therefore be operated by hand or by an automated mechanism, rather than being breath operated. Further, the pinch valve may be replaced, for example, by a sliding gate valve arrangement.

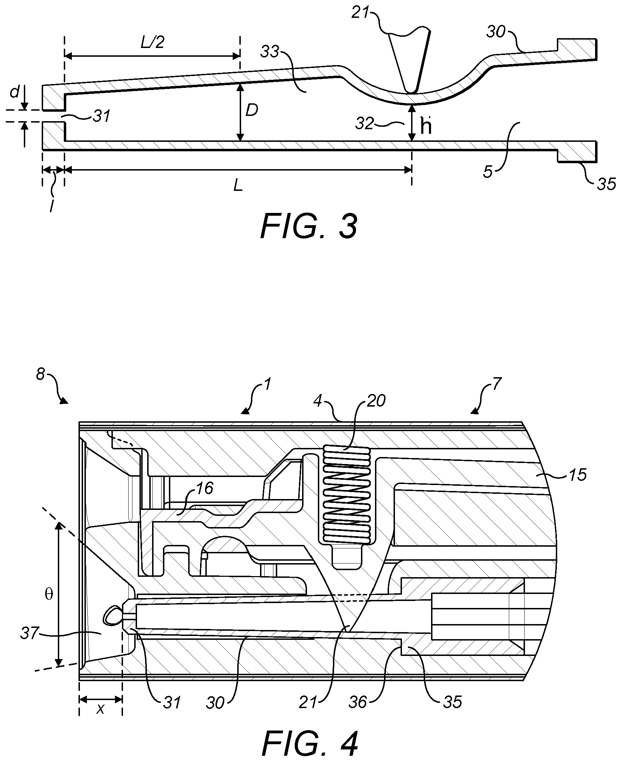

The present invention is described with reference to Fig. 3 which discloses a pinched pinch tube in which the outlet orifice is applied. The pinch tube 30 has an exit orifice 31 at its downstream end and is pinched closed by jaw 21 in a region in the vicinity of the opposite end. The point where it is pinched closed represents the valve outlet orifice 32 which has a maximum height h measured in the direction of opening when the valve is fully open. To the right of this pinch point is the reservoir 5 containing the inhalable composition. Part of the reservoir is made up by the right-hand portion of the pinch tube 30, and the remainder is made up by the device housing as described above.

Between the valve outlet orifice 32 and the exit orifice 31 is expansion chamber 33. This has an axial length L and an internal diameter D which is measured halfway along the expansion chamber. The exit orifice 31 has a length 1 and a diameter d.

Fig. 4 shows how the pinch tube 30 is incorporated into the inhaler. Where appropriate, the same reference numerals have been used for the same components to designate components equivalent to the same components described above in relation to Figs. 1 and 2. An annular rim 35 at the opposite to the exit orifice 31 engages with a step 36 within the body of the housing 1 to maintain the pinch tube 30.

The closure of the tube is shown schematically in Fig. 4 in that, rather than penetrating the wall of the pinch tube 30, the jaw 21 will, in fact, simply compress the tube as described above.

The axial distance x between the end of the exit orifice 31 and the outlet end 8 is 1.4 mm. Between the exit orifice 31 and the outlet end 8 is a flared flow path 37 which is flared with an angle θ of 51.1°. This short distance and wide angle of flow path prevents as far as possible any impingement of the composition leaving the outlet orifice 31 on the body of the inhaler.

The inhaler has a reservoir volume of approximately 1ml maintained at a pressure of 600kPa. The opening height (h) is 0.1mm in the fully opened position. The length (L) is 7.4mm and the diameter (D) is 0.94mm. The exit orifice has a length (1) of 0.5mm and an internal diameter (d) of 0.2mm.

## Claims

1. An inhaler comprising a reservoir (5) of pressurised inhalable composition, an outlet valve (7) to control the flow of composition from the reservoir (5), the valve outlet orifice (32) having a maximum height h, measured in the direction of opening when fully opened; an expansion chamber (33) downstream of the valve (7) having a length L and diameter D measured half way along the expansion chamber (33); and an exit orifice (31) at the downstream end of the expansion chamber (33), the exit orifice (31) having a length 1 and a diameter d; **characterised in that**:
0.1 < h/d < 1.0
0.05 < h/D < 0.25
1 < D/d < 10
5 < L/D < 15
2 < 1/d < 3
wherein the exit orifice (31) is less than 3mm from an outlet end (8) of the inhaler.

2. An inhaler according to claim 1, wherein h/d is between 0.2 and 0.9 and more preferably substantially 0.5.

3. An inhaler according to claim 1 or claim 2, wherein h/D is between 0.07 and 0.2 and more preferably between 0.08 and 0.15.

4. An inhaler according to claim 3 wherein h/D is between 0.092 and 0.143 and most preferably 0.11.

5. An inhaler according to any one of the preceding claims, wherein D/d is between 2 and 7 and more preferably between 3 and 6.

6. An inhaler according to claim 5 wherein D/d is between 4 and 5 and most preferably substantially 4.5.

7. An inhaler according to any one of the preceding claims, wherein L/D is between 6 and 13, preferably between 7 and 10 and more preferably, between 7.65 and 8.30.

8. An inhaler according to any one of the preceding claims, wherein the expansion chamber (33) tapers from the valve outlet orifice (32).

9. An inhaler according to claim 8, wherein the taper has an included angle of between 0 and 30º, preferably between 9 and 10º and most preferably substantially 2º.

10. An inhaler according to any one of the preceding claims, wherein the outlet valve (7) is a pinch valve in which a valve element (21) pinches a deformable tube (30) with the outlet orifice height h representing the maximum opening height at the pinch point.

11. An inhaler according to claim 10, wherein the deformable tube (30) also provides the expansion chamber (33) and the exit orifice (31).

12. An inhaler according to any one of the preceding claims, wherein the outlet valve (7) is a breath operated valve.

13. An inhaler according to any one of the preceding claims, wherein the inhaler is a simulated cigarette.

14. An inhaler according to any one of the preceding claims, wherein the inhalable composition contains nicotine and a propellant.

15. An inhaler according to any preceding claim, wherein between the exit orifice (31) and the outlet end (8) of the inhaler, there is a flared flow path with an angle of at least 10°.

## Patentansprüche

1. Inhalator, der ein Reservoir (5) mit einer unter Druck stehenden inhalierbaren Zusammensetzung, ein Auslassventil (7) zum Steuern der Menge der aus dem Reservoir (5) strömenden Zusammensetzung, wobei die Ventilauslassöffnung (32) bei Messung in Richtung der vollständig geöffneten Öffnung eine maximale Höhe h aufweist, eine stromabwärts des Ventils (7) angeordnete Expansionskammer (33), die eine Länge L und in der Mitte der Expansionskammer (33) einen Durchmesser D besitzt, und eine sich am stromabwärts gelegenen Ende der Expansionskammer (33) befindende Austrittsöffnung (31) aufweist, wobei die Austrittsöffnung (31) eine Länge 1 und einen Durchmesser d besitzt, **dadurch gekennzeichnet, dass**:
0,1 < h/d < 1,0
0,05 < h/D < 0,25
1 < D/d < 10
5 < L/D < 15
2 < 1/d < 3
wobei die Austrittsöffnung (31) weniger als 3 mm von einem Auslassende (8) des Inhalators entfernt ist.

2. Inhalator nach Anspruch 1, wobei h/d zwischen 0,2 und 0,9 liegt und bevorzugt in etwa 0,5 beträgt.

3. Inhalator nach Anspruch 1 oder 2, wobei h/D zwischen 0,07 und 0,2 und bevorzugt zwischen 0,08 und 0,15 liegt.
0184-76414EP-AS/MM

4. Inhalator nach Anspruch 3, wobei h/D zwischen 0,092 und 0,143 liegt und bevorzugter 0,11 beträgt.

5. Inhalator nach einem der vorhergehenden Ansprüche, wobei D/d zwischen 2 und 7 und bevorzugt zwischen 3 und 6 liegt.

6. Inhalator nach Anspruch 5, wobei D/d zwischen 4 und 5 liegt und bevorzugter in etwa 4,5 beträgt.

7. Inhalator nach einem der vorhergehenden Ansprüche, wobei L/d zwischen 6 und 13, vorzugsweise zwischen 7 und 10 und bevorzugt zwischen 7,65 und 8,30 liegt.

8. Inhalator nach einem der vorhergehenden Ansprüche, wobei die Expansionskammer (33) sich nach der Ventilauslassöffnung (32) verjüngt.

9. Inhalator nach Anspruch 8, wobei die Verjüngung einen Winkel einschließt, der zwischen 0° und 30° liegt, vorzugsweise zwischen 9° und 10° liegt, und bevorzugter in etwa 2° beträgt.

10. Inhalator nach einem der vorhergehenden Ansprüche, wobei es sich bei dem Auslassventil (7) um ein Quetschventil handelt, bei dem ein Ventilelement (21) einen verformbaren Schlauch (30) quetscht, wobei die Höhe h der Auslassöffnung einer maximalen Öffnungshöhe an der Quetschposition entspricht.

11. Inhalator nach Anspruch 10, wobei auch die Expansionskammer (33) und die Austrittsöffnung (31) von dem verformbaren Schlauch (30) gebildet werden.

12. Inhalator nach einem der vorhergehenden Ansprüche, wobei es sich bei dem Auslassventil (7) um ein atmungsgesteuertes Ventil handelt.

13. Inhalator nach einem der vorhergehenden Ansprüche, wobei es sich bei dem Inhalator um eine simulierte Zigarette handelt.

14. Inhalator nach einem der vorhergehenden Ansprüche, wobei die inhalierbare Zusammensetzung Nikotin und ein Treibmittel enthält.

15. Inhalator nach einem der vorhergehenden Ansprüche, wobei sich zwischen der Austrittsöffnung (31) und dem Auslassende (8) des Inhalators ein sich aufweitender Strömungspfad befindet, dessen Winkel zumindest 10° beträgt.

## Revendications

1. Inhalateur qui comprend un réservoir (5) de composition inhalable pressurisée, une soupape de sortie (7) destinée à contrôler le flux de composition qui sort du réservoir (5), l'orifice de la soupape de sortie (32) ayant une hauteur maximale h, mesurée dans la direction d'ouverture en position d'ouverture maximale ;
une chambre d'expansion (33) en aval de la soupape (7), qui possède une longueur L et un diamètre D mesurés à mi-chemin le long de la chambre d'expansion (33) ; et
un orifice de sortie (31) au niveau de l'extrémité aval de la chambre d'expansion (33), l'orifice de sortie (31) ayant une longueur l et un diamètre d ; **caractérisé en ce que** :
0,1 < h/d < 1
0,05 < h/D < 0,25
1 < D/d < 10
5 < L/D < 15
2 < l/d < 3
dans lequel l'orifice de sortie (31) se trouve à moins de 3 mm par rapport à une extrémité de sortie (8) de l'inhalateur.

2. Inhalateur selon la revendication 1, dans lequel h/d est compris entre 0,2 et 0,9, et est de préférence égal à 0,5.

3. Inhalateur selon la revendication 1 ou la revendication 2, dans lequel h/D est compris entre 0,07 et 0,2, et de préférence entre 0,08 et 0,15.

4. Inhalateur selon la revendication 3 dans lequel h/D est compris entre 0,092 et 0,143, et est de préférence égal à 0,11.

5. Inhalateur selon l'une quelconque des revendications précédentes, dans lequel D/d est compris entre 2 et 7, et de préférence entre 3 et 6.

6. Inhalateur selon la revendication 5, dans lequel D/d est compris entre 4 et 5, et est de préférence égal à 4,5.

7. Inhalateur selon l'une quelconque des revendications précédentes, dans lequel L/D est compris entre 6 et 13, de préférence entre 7 et 10, et de préférence entre 7,65 et 8,30.

8. Inhalateur selon l'une quelconque des revendications précédentes, dans lequel la chambre d'expansion (33) est effilée à partir de l'orifice de la soupape de sortie (32).

9. Inhalateur selon la revendication 8, dans lequel la partie effilée présente un angle compris entre 0 et 30°, de préférence entre 9 et 10,° et de préférence égal à 2°.

10. Inhalateur selon l'une quelconque des revendications précédentes, dans lequel la soupape de sortie (7) est une soupape à étranglement dans laquelle un élément de soupape (21) étrangle un tube déformable (30), avec la hauteur h de l'orifice de sortie qui représente la hauteur d'ouverture maximale au niveau point d'étranglement.

11. Inhalateur selon la revendication 10, dans lequel le tube déformable (30) alimente également la chambre d'expansion (33) et l'orifice de sortie (31).

12. Inhalateur selon l'une quelconque des revendications précédentes, dans lequel la soupape de sortie (7) est une soupape qui réagit à la respiration.

13. Inhalateur selon l'une quelconque des revendications précédentes, dans lequel l'inhalateur est une cigarette simulée.

14. Inhalateur selon l'une quelconque des revendications précédentes, dans lequel la composition inhalable contient de la nicotine et un gaz propulseur.

15. Inhalateur selon l'une quelconque des revendications précédentes, dans lequel il existe un trajet d'écoulement évasé avec un angle d'au moins 10° entre l'orifice de sortie (31) et l'extrémité de sortie (8) de l'inhalateur.
